# EUROPÄISCHE PATENTSCHRIFT

(11) **EP 1 313 443 B1**
(45) Veröffentlichungstag und Bekanntmachung des Hinweises auf die Patenterteilung: **28.12.2005**
(21) Anmeldenummer: 01960716.7
(22) Anmeldetag: 30.08.2001
(51) Int. Cl.: A61K 7/48, A61K 7/027, A61K 7/035, A61K 7/00

(54) **HOCHVISKOSES KOSMETISCHES PRODUKT**
HIGHLY VISCOUS COSMETIC PRODUCT
PRODUIT COSMETIQUE FORTEMENT VISQUEUX

(30) Priorität: 31.08.2000 DE 10044062
(43) Veröffentlichungstag der Anmeldung: 28.05.2003
(73) Patentinhaber: Coty B.V., 2031 CC Haarlem (NL)
(72) Erfinder: CERNASOV, Domnica, Ringwood, NJ 07456 (US); KOLMAN, Brian, Hillsborough, NJ 08876 (US); GUERRERO, Alberto, Flanders, NJ 07836 (US); ATTLE, Steve, San Diego, CA 92111 (US); MACCHIO, Ralph, Sparta, NJ 07971 (US)
(74) Vertreter: Walter, Wolf-Jürgen
(86) Internationale Anmeldenummer: PCT/EP2001/010017
(87) Internationale Veröffentlichungsnummer: WO 2002/017875

(56) Entgegenhaltungen:
- EP-A- 0 611 207
- EP-A- 0 923 930
- FR-A- 2 794 997
- FR-A- 2 795 000

## Beschreibung

Die Erfindung betrifft ein hochviskoses kosmetisches Produkt mit neuartiger Struktur auf Wachs- und Gelbasis.

Aus der EP-A-976382 sind kosmetische oder pharmazeutische Pflaster bekannt, die eine wäßrige Phase aufweisen und in einem hydrophilen Gelsystem das Gel Gellan Gum neben einem Hydrokolloid enthalten.

Die EP 985410 betrifft eine flüssige oder cremige kosmetische Zusammensetzung mit Gellan gum und einem stabilisierenden Carboxyvinylpolymeren.

Aufgabe der vorliegenden Erfindung ist es, ein neues kosmetisches Produkt mit bisher nicht bekannter Struktur bereitzustellen, das Wachse und Gele enthält.

Erfindungsgemäß bereitgestellt wird ein Kosmetikum, das eine Kombination der Struktur eines Gels, einer Emulsion und eines Wachses darstellt und dabei folgende Komponenten enthält:
0,01 - 8 Gew-% von wenigstens zwei Gelbildnern, wovon der Anteil des Gelbildners Gellan Gum im Bereich von 0,01 - 3 Gew-% liegt,
0,01 - 10 Gew-% eines Feuchthaltemittels,
0,1 - 2 Gew-% eines Härtungsmittels für die Struktur des Gels, enthaltend anorganische, in Wasser wenigstens teilweise lösliche Salze,
0,1 - 10 Gew-% natürliche oder synthetische kosmetische Wachse oder Gemische davon,
10 - 90 Gew-% Emulsionsbildner, ausgewählt unter kosmetischen Ölen, Estern, Gemischen von Ölen und Estern, und Wasser,
0,01 - 15 Gew-% eines anionischen oder nichtionischen Emulgators oder eines Gemisches davon, und
4 bis 30 Gew-% eines Pigments oder Pigmentgemisches,
wobei das Kosmetikum eine Härte im Bereich von 15 bis 66 hat, gemessen mit einem Penetrometer, Konus 12 g, und wobei alle Prozentangaben auf die Gesamtmasse des Produktes bezogen sind.

Das neuartige Kosmetikum wird auf Grund der Struktur als pigmetiertes Produkt hergestellt, das insbesondere farbige Pigmente wie Eisenoxide enthält. Während bei üblichen Gelen des Standes der Technik, wie das in der EP-A-976382 oder in EP 985410 offenbarte Gel, Pigmente wie Eisenoxide die Gelstruktur brechen, lassen sich in das erfindungsgemäße Kosmetikum übliche Pigmente problemlos bis zu Konzentrationen von 30 Gew-% einarbeiten. Bevorzugt sind 4-24, insbesondere 5 bis 15 Gew-% Pigmente. Zu diesen Pigmenten gehören Eisenoxide, natürliche Aluminiumsilicate wie Ocker, Titandioxid, Glimmer, Kaolin, manganhaltige Tone wie Umbra und roter Bolus, Calciumcarbonat, Talkum, Silica, Glimmer-Titanoxid, Glimmer-Titanoxid-Eisenoxid, Glimmer-Titanoxid-organischer Farbstoff, Bismuthoxychlorid, Nylonkügelchen, Keramikkügelchen, expandierte und nichtexpandierte synthetische Polymerpulver, pulverförmige natürliche organische Verbindungen wie gemahlene Festalgen, gemahlene Pflanzenteile, verkapselte und unverkapselte Getreidestärken und Gemische davon.

Weiterhin ist für das erfindungsgemäße Gel der sehr hohe Penetrationswert (Härte) hervorzuheben. Aus praktischen Gründen wird bevorzugt, besser mit dem Penetrationswert zu arbeiten als mit der Viskosität. Bevorzugte Bereiche des Penetrationswertes sind für dünne Stifte (z.B. 5-15 mmm) wie Lippenstifte, Fettstifte oder Makeup-Stifte Werte von 15 bis 30, für dickere Stifte (z.B. >15 mm) wie Grundierungsstifte (Foundation sticks) 31 bis 66 und für Kompaktformen (compacts) 42 bis 52. Die Messung des Penetrationswertes erfolgt mit einem Penetrometer, z.B. Lab-Line (hergestellt von Lab-Line Instruments Inc., USA), Konus 12 g, Gewichte 50 g. Ein Ring mit folgenden Maßen: Höhe 3,5 cm, äußerer Durchmesser 3,7 cm, innerer Durchmesser 3,4 cm wird auf ein Paraffinpapier gelegt. Eine Probe wird auf Fließtemperatur erwärmt, in den Ring gegossen und 12-24 Stunden in kontrollierter Umgebung bei 25 °C abkühlen gelassen. Danach wird das Paraffinpapier vom Ring entfernt auf dieser Seite die Messung vorgenommen.

Der Konus wird im Penetrometerkopf befestigt, das Gewicht dem Belastungsstab zugefügt und der Meßkopf abgesenkt, so daß die Spitze des Konus gerade so die Probenoberfläche berührt. Dann wird der Druckstempel vorsichtig über einen Auslösemechanismus freigegeben und für 5 Sekunden ein Eindringen des Konus in die Probe ermöglicht. Danach wird am Meßstab der ermittelte Wert abgelesen. Es werden 4 Meßwerte von verschiedenen Stellen der Probe genommen und ein Durchschnittswert gebildet.

Als Härtungsmittel für die Struktur des Gels enthält die Zusammensetzung vorzugsweise anorganische Salze, die aus der Gruppe ausgewählt sind, die aus Natrium-, Kalium-, Magnesium-, Calcium-, Mangan(II)-, Barium-, Eisen(II)-, Eisen(III)salzen und Gemischen davon besteht.

Das erfindungsgemäße Kosmetikum enthält weiterhin natürliche oder synthetische Wachse oder deren Gemische, vorzugsweise im Bereich von 0,1 - 10 Gew- %. Als Wachse können eingesetzt werden natürliche pflanzliche Wachse, tierische Wachse, natürliche und synthetische Mineralwachse und synthetische Wachse. Dazu gehören beispielsweise Carnaubawachs, Candellilawachs, Bienenwachs, Wollwachs, Paraffin, Ceresin, Ozolcerit, Silicone, Polyethylenglycol- oder -glycolesterwachse, Montanwachs und Gemische davon.

Der erfindungsgemäß eingesetzte Gelbildner Gellan gum ist ein Polysaccharid, das durch gesteuerte Fermentation von Pseudomonas elodea hergestellt wird. Er ist als multifunktionelles Hydrokolloid bisher insbesondere in der Nahrungsmittelindustrie eingesetzt worden. Die Gelierung erfolgt durch Kationen, wie Natrium-, Kalium-, Magnesium, Calcium und Wasserstoffionen, wie sie in vielen kosmetischen Produkten enthalten sind oder zielgerichtet zugesetzt werden. Die Ionenkonzentration beeinflußt die Gelfestigkeit und kann vom Fachmann entsprechend eingestellt werden. Ein bevorzugtes Gellan Gum ist Kelcogel® von NutraSweet, San Diego, CA 92123-1718, USA.

Weitere Gelbildner können in Kombination mit Gellan Gum für das erfindungsgemäße Kosmetikum eingesetzt werden, wie Carbomer, Xanthangummi, Carrageenan, Akaziengummi, Guargummi, Agar-Agar, Alginate und Tylosen, Magnesiumaluminiumsilicat, Carboxymethylcellulose, Hydroxyethylcellulose, bestimmte Polyacrylate, Polyvinylalkohol, Polyvinylpyrrolidon, Montmorillonit und Gemische davon. Dabei liegt der Anteil von Gellan Gum im Bereich von 0,01-3 Gew-%, und der damit kombinierte Gelbildner, wie vorzugsweise Xanthangummi, im Bereich von 0,01 bis 8 Gew-%. Eine Kombination von Gellan Gum mit einem oder mehreren anderen Gelbildner ist vorteilhaft, um die Gelhärte in ein gewünschtes Gleichgewicht zu bringen.

Als dritter Hauptbestandteil neben dem Wachs und dem Gel tritt eine Emulsion auf. Die Emulsion ist eine feste Emulsion (Granulat, Paste). Sie wird beispielsweise gebildet mit einem üblichen kosmetischen Öl oder einem Ester oder Ether, und stellt zusammen mit Wasser eine O/W- oder W/O-Emulsion dar.

Geeignete öle und Ester sind beispielsweise Mineralöl, hydriertes Polyisobuten, synthetisches oder aus Naturprodukten hergestelltes Squalan; kosmetische Ester oder Ether, die verzweigt oder unverzweigt, gesättigt oder ungesättigt sein können; pflanzliche Öle; oder Gemische zweier oder mehrerer davon.

Besonders geeignet Öle sind beispielsweise Mineralöle, Hydrogenated Polyisobuten, Polyisopren, Squalane, Tridecyltrimellitat, Trimethylpropan-triisostearat, Isodecylcitrat, Neopentylglycol-diheptanoat, PPG-15-stearylether sowie pflanzliche öle, wie Calendulaöl, Jojobaöl, Avocadoöl, Macadamianußöl, Rizinusöl, Weizenkeimöl, Traubenkernöl, Kukuinußöl, Distelöl, Nachtkerzenöl, Safloröl oder ein Gemisch mehrerer davon.

Als Ester oder Ether sind zum Beispiel geeignet (INCI-Namen): Caprylic Capric Triglyceride, Cholesteryl Behenyl Dodecyl Lauroyl Glutamate, Behenyl Erucate, Dipentaerythrityl hexacaprilate/hexacaprate/tridecyl trimellitate/tridecyl stearate/neopentyl glycol dicaprylate dicaprate, Propylene glycol dioctanoate 5, Propylene glycol dicaprylate 2,30 dicaprate, Tridecyl stearate/neopentyl glycol dicaprylate dicaprate/tridecyl trimellitate, Neopentyl glycol dioctanoate, Isopropyle myristate, Diisopropyl dimer dilinoleate, Trimethylpropane triisostearate, Myristyl ether, Stearyl ether, Butyl ether, Dicaprylyl ether, PPG1-PEG9 Lauroyl glycol ether, PPG15 Stearyl ether, PPG14 Butyl ether, Fomblin HC25.

Als Emulgatoren können eingesetzt werden Anlagerungsprodukte von 2-30 Mol Ethylenoxid an lineare C₈-C₂₂-Fettalkohole, an C₁₂-C₂₂-Fettsäuren und an C₈-C₁₅-Alkylphenole; C₁₂-C₂₂-Fettsäuremono- und -diester von Anlagerungsprodukten von 1-30 Mol Ethylenoxid an Glycerin; oder Anlagerungsprodukte von 2-15 Mol Ethylenoxid an Ricinusöl; Ester von C₁₂-C₂₂-Fettsäuren und Glycerin, Polyglycerin, Pentaerythrit, Zuckeralkohole (z.B. Sorbit), Polyglucoside (z.B. Cellulose); Polyalkylenglycole; Wollwachsalkohole; Copolymere von Polysiloxan-Polyalkylpolyether, Lecithin. Bevorzugt sind z.B. Tween 20® oder Span 60®.

Für die Struktur des Produktes weiterhin notwendig sind Härtungsmittel wie beispielsweise Behenylerucat oder wachse, wie Montanwachs, Carnaubawachs, Candelillawachs, Ozokerit und deren Gemische im Bereich von 0,1-10 Gew-%.

Geeignete Feuchthaltemittel sind beispielsweise Glycerin, Butylenglycol, Propylenglycol, Algenextrakte, NaPCA, Aloe vera, Allantoin, Panthenol.

Das erfindungsgemäße Kosmetikum kann weitere Wirkstoffe enthalten, wie anorganische und organische Lichtschutzmittel, Radikalfänger, Vitamine, Enzyme, pflanzliche Wirkstoffe, Algenextrakte, Polymere, Melanin, Antioxidationsmittel, entzündungswidrige natürliche Wirkstoffe. Weiterhin kann es übliche Hilfsstoffe wie Propylenglycol, Lecithin, Caprylic/Capric Triglyceride, Diisopropyl Dimer Dilinolate, Neopentyl Glycol Dicaprate, Sorbitan Stearate, Acrylates/Carbamate Copolymer, Kationen, Saccharide Isomerate, Konservierungsstoffe, Parfüme usw. enthalten.

Das erfindungsgemäße Kosmetikum hat eine besondere Textur, da es eine ähnliche Erscheinungsform wie ein Preßpuder hat, wie eine Flüssigkeit angewendet wird und dabei den Aspekt eines "heiß gegossenen" Produktes zeigt.

Es kann z.B. als frei stehender Stift z.B. für Lippenstifte, Lippenglanz, Lippenbalsam; als Gießmaterial für verschiedene Behälter beispielsweise für Schminke, Grundierungen, Make-up; als Patches (Pflaster) zur lokalen Behandlung bestimmter Nagtstellen; sowie als Farbstift (crayons) eingesetzt werden.

Die Herstellung des erfindungsgemäßen Kosmetikums erfolgt in der Weise, daß zuerst das Gel gebildet wird durch Zugabe des Gelbildners in bei einer Temperatur im Bereich von 75 bis 80 °C. Die separat hergestellte ölphase, in der Wachse und Ester enthalten sind sowie weitere Bestandteile wie Feuchthaltemittel, weitere Härter, Emulgatoren und bestimmte öle, wird dann bei 75 bis 80 °C unter Rühren hinzugesetzt.

Schließlich wird das Gemisch bei 75 bis 80 °C homogenisiert und nach Abkühlen auf 70 bis 75 °C bei Temperaturen zwischen 65 und 80 °C in Formen oder Tiegel gegossen.

Die Erfindung soll nachstehend durch Beispiele näher erläutert werden. Alle Angaben erfolgen in Gewichtsprozent, sofern nichts anderes angegeben ist.

### Beispiel 1 Kühlender Lippenstift

| **Phase A** | |
|---|---|
| Wasser | bis 100 |
| Gellan gum | 0,8 |
| Butylenglycol | 2 |
| Xantham gum | 0,3 |

| **Phase B** | |
|---|---|
| Polysorbate | 2 |

| **Phase C** | |
|---|---|
| Glycerin | 11 |
| Farbpigmente | 6 |
| Silica | 1 |
| Nylon-12 | 3 |
| Talkum | 6 |

| **Phase D** | |
|---|---|
| Candelillawachs | 4,5 |
| Ozokerite | 0,5 |
| Lecithin | 1,5 |
| Konservierungsmittel | 0,5 |
| Behenylerucat | 0,7 |
| Diisopropyl Dimer Dilinoleate | 2 |
| Neopentyl Glycol Dicaprate | 2 |
| Sorbitan Stearate | 2 |

Die Gelbildner werden mit Wasser verrührt und zusammen mit den anderen Komponenten der Phase A auf etwa 75 °C erhitzt. Die jeweils separat gemischten Phasen B, C und D werden Phase A unter Rühren hinzugegeben. Das Gemisch wird bei etwa 75 °C homogenisiert, auf etwa 70 °C abgekühlt und in Lippenstiftformen gegossen. Die Lippenstifte hatten einen Penetrationswert von 21.

### Beispiel 2 Kühlender Grundierungsstift

| **Phase A** | |
|---|---|
| Wasser | bis 100 |
| Gellan gum | 0,5 |
| Butylenglycol | 2 |
| Xantham gum | 0,3 |
| Natriumchlorid | 0,2 |

| **Phase B** | |
|---|---|
| Polysorbate | 2 |

| **Phase C** | |
|---|---|
| Glycerin | 11 |
| Farbpigmente | 8,5 |
| Silica | 1,5 |
| Bornitrid | 2 |
| Talkum | 4 |

| **Phase D** | |
|---|---|
| Sorbitan Stearate | 2,5 |
| Candelillawachs | 4,5 |
| Ozokerite | 0,8 |
| Ceresinwachs | 1,5 |
| Lecithin | 1 |
| Konservierungsmittel | 0,5 |
| Caprylic Capric Triglyceride | 3,5 |
| Diisopropyl Dimer Dilinoleate | 2 |
| Neopentyl Glycol Dicaprate | 3 |

Die Herstellung erfolgte ähnlich wie im Beispiel 1. Die in Formen gegossenen Stifte hatten einen Penetrationswert von 55.

### Beispiel 3 Kompakt-Schattierungsmittel

| **Phase A** | |
|---|---|
| Wasser | bis 100 |
| Gellan gum | 0,7 |
| Butylenglycol | 3 |
| Xantham gum | 0,3 |
| Allantoin | 0,01 |

| **Phase B** | |
|---|---|
| Polysorbate | 2 |

| **Phase C** | |
|---|---|
| Glycerin | 10 |
| Farbpigmente | 6 |
| Silica | 1 |
| Bariumsulfat | 2 |
| Talkum | 5 |
| Nylon-12 | 3 |

| **Phase D** | |
|---|---|
| Candelillawachs | 3,5 |
| Ozokerite | 0,5 |
| Lecithin | 1,5 |
| Behenyl Erucate | 0,5 |
| Caprylic Capric Triglyceride | 3 |
| Diisopropyl Dimer Dilinoleate | 2 |
| Neopentyl Glycol Dicaprate | 3 |
| Sorbitan Stearate | 2,5 |

| **Phase E** | |
|---|---|
| Algenextrakt/Tocopherol/Retinylpalmitat | 0,1 |
| Konservierungsmittel | 0,2 |
| Parfüm | 0,3 |

Die Herstellung erfolgte ähnlich wie im Beispiel 1. Das in Formen gegossene Kompakt-Mittel hatte einen Penetrationswert von 47.

## Patentansprüche

1. Hochviskoses kosmetisches Produkt, **gekennzeichnet durch** die Kombination der Struktur eines Gels, einer Emulsion und eines Wachses, wobei die Kombination enthält
0,01 - 8 Gew-% von wenigstens zwei Gelbildnern, wovon der Anteil des Gelbildners Gellan Gum im Bereich von 0,01 - 3 Gew-% liegt,
0,01 - 10 Gew-% eines Feuchthaltemittels,
0,1 - 2 Gew-% eines Härtungsmittels für die Struktur des Gels, enthaltend anorganische, in Wasser wenigstens teilweise lösliche Salze,
0,1 - 10 Gew-% natürliche oder synthetische kosmetische Wachse oder Gemische davon,
10 - 90 Gew-% Emulsionsbildner, ausgewählt unter kosmetischen Ölen, Estern, Gemischen von Ölen und Estern, und Wasser,
0,01 - 15 Gew-% eines anionischen oder nichtionischen Emulgators oder eines Gemisches davon,
4 bis 30 Gew-% eines Pigments oder Pigmentgemisches,
wobei das Produkt einen Penetrationswert im Bereich von 15 bis 66 hat, gemessen mit einem Lab-line Penetrometer, Konus 12 g, und wobei alle Prozentangaben auf die Gesamtmasse des Produktes bezogen sind.

2. Kosmetisches Produkt nach Anspruch 1, **dadurch gekennzeichnet, daß** es 4 bis 24 Gew-% Pigmente enthält.

3. Kosmetisches Produkt nach Anspruch 2, **dadurch gekennzeichnet, daß** es 5 bis 15 Gew-% Pigmente enthält.

4. Kosmetisches Produkt nach Anspruch 1, **dadurch gekennzeichnet, daß** es als Pigmente Eisenoxide, natürliche Aluminiumsilicate, Titaniumoxid, Glimmer, Kaolin, manganhaltige Tone, Talkum, Nylonkügelchen, Siliciumdioxid und Gemische davon enthält.

5. Kosmetisches Produkt nach Anspruch 1, **dadurch gekennzeichnet, daß** der Anteil der natürlichen oder synthetischen Wachse oder deren Gemische im Bereich von 0,1 - 10 Gew- % liegt.

6. Kosmetisches Produkt nach Anspruch 1, **dadurch gekennzeichnet, daß** die Wachse ausgewählt sind unter Paraffinwachs, Carnaubawachs, Montanwachs, Candelillawachs, Ozokerit und Gemischen davon.

7. Kosmetisches Produkt nach Anspruch 1, **dadurch gekennzeichnet, daß** neben Gellan Gum weiterhin als Gelbildner vorhanden sind solche aus der Gruppe, ausgewählt unter Carbomer, Xanthangummi, Carrageenan, Akaziengummi, Guargummi, Agar-Agar, Alginate, PVA, PVP und Gemischen davon.

8. Kosmetisches Produkt nach Anspruch 1, **dadurch gekennzeichnet, daß** die anorganischen Salze aus der Gruppe ausgewählt sind, die aus Natrium-, Kalium-, Magnesium-, Calcium-, Mangan(II)-, Barium-, Eisen(II)-, Eisen(III) salzen und Gemischen davon besteht.

9. Kosmetisches Produkt nach Anspruch 1, **dadurch gekennzeichnet, daß** das Produkt ein Stift ist mit einem Durchmesser von 5-15 mm und einem Penatrionswert von 15 bis 30.

10. Kosmetisches Produkt nach Anspruch 1, **dadurch gekennzeichnet, daß** das Produkt ein Stift ist mit einem Durchmesser >15 mm und einem Penatrionswert von 31 bis 66.

11. Kosmetisches Produkt nach Anspruch 1, **dadurch gekennzeichnet, daß** das Produkt eine Kompakt-Grundierung ist mit einem Penatrionswert von 42 bis 52.

## Claims

1. Highly viscous cosmetic product which comprises the combination of the structure of a gel, an emulsion, and a wax,
wherein said combination comprises 0.01 - 8 weight% of at least two gel formers wherein the share of the Gellan gum gel former ranges between 0.01 - 3 weight percent,
0.01 - 10 weight% of a moisturizer,
0.1 - 5 weight% of a hardening agent for the structure of the gel comprising inorganic salts which are at least partly soluble in water,
0.1 - 10 weight% of natural or synthetic cosmetic waxes or mixtures thereof,
10 - 90 weight% of an emulsion former selected among cosmetic oils, esters, mixtures of oils and esters, and water,
0.01 - 15 weight% of an anionic or non-ionic emulsifier or a mixture thereof, and
4 - 30 weight% of a pigment or mixture of pigments,
wherein the product has a Penetration value ranging from 15 to 66, measured by a penetrometer Lab-line, 12 gr. cone, and
wherein all percents are related to the entire weight of the product.

2. Cosmetic product according to Claim 1, which comprises 4 to 24 weight% of pigments.

3. Cosmetic product according to Claim 2, which comprises 5 to 15 weight% of pigments.

4. Cosmetic product according to Claim 1, which comprises as pigments iron oxides, natural aluminium silicates, titanium oxide, mica, kaolin, manganese-containing clays, talc, nylon beads, silicon dioxide, and mixtures thereof.

5. Cosmetic product according to Claim 1, wherein the share of the natural or synthetic waxes or mixtures thereof ranges between 0.1 - 10 weight%.

6. Cosmetic product according to Claim 1, wherein the waxes are selected among paraffin wax, carnauba wax, montan wax, candelilla wax, ozokerite, and mixtures thereof.

7. Cosmetic product according to Claim 1, wherein apart from Gellan gum, further gel formers of the group are present, being selected among carbomer, xanthan rubber, carrageenan, acacine, guar rubber, agar-agar, alginates, PVA, PVP, and mixtures thereof.

8. Cosmetic product according to Claim 1, wherein the inorganic salts are selected from the group consisting of sodium, magnesium, potassium, calcium, manganous(II), barium, ferrous, ferric salts and mixtures thereof.

9. Cosmetic product according to Claim 1, wherein the product is a stick of 5-15 mm diameter with a penetration value of 15 to 30.

10. Cosmetic product according to Claim 1, wherein the product is a stick of >15 mm diameter with a penetration value of 31 to 6.

11. Cosmetic product according to Claim 1, wherein the product is a compact foundation with a penetration value of 42 to 52.

## Revendications

1. Produit cosmétique fortement visqueux, **caractérisé par** la combinaison de structure d'un gel, d'une émulsion et d'une cire, la combinaison contenant
0,01 - 8 % en poids d'au moins deux gélifiants, dont la proportion en gélifiant gomme de gellan se trouve dans le domaine de 0,01 - 3 % en poids,
0,01 - 10 % en poids d'un humectant,
0,1 - 2 % en poids d'un durcisseur pour la structure du gel, contenant des sels inorganiques, se dissolvant au moins partiellement dans l'eau,
0,1 - 10 % en poids de cires cosmétiques naturelles ou synthétiques ou de mélanges de celles-ci,
10 - 90 % en poids de formateur d'émulsion, choisi parmi des huiles cosmétiques, des esters, des mélanges d'huiles et d'esters, et de l'eau,
0,01 - 15 % en poids d'un émulsifiant anionique ou non ionique ou d'un mélange de ceux-ci,
4 à 30 % en poids d'un pigment ou d'un mélange de pigments, le produit ayant une valeur de pénétration dans le domaine de 15 à 66, mesuré avec un pénétromètre Lab-Line, cône 12 g, et toutes les données en pourcentage étant rapportées à la masse totale du produit.

2. Produit cosmétique selon la revendication 1, **caractérisé en ce qu'**il contient 4 à 24 % en poids de pigments.

3. Produit cosmétique selon la revendication 2, **caractérisé en ce qu'**il contient 5 à 15 % en poids de pigments.

4. Produit cosmétique selon la revendication 1, **caractérisé en ce qu'**il contient en tant que pigments de l'oxyde de fer, des silicates d'aluminium naturels, de l'oxyde de titane, du mica, du caolin, des argiles contenant du manganèse, du talc, des sphérules de nylon, du dioxyde de silicium et des mélanges de ceux-ci.

5. Produit cosmétique selon la revendication 1, **caractérisé en ce que** la proportion de cires naturelles ou synthétiques ou des mélanges de celles-ci se trouve dans le domaine de 0,1 à 10 % en poids.

6. Produit cosmétique selon la revendication 1, **caractérisé en ce que** les cires sont choisies parmi la cire de paraffine, la cire de carnauba, la cire de lignite, la cire de candelilla, l'ozocérite et des mélanges de ceux-ci.

7. Produit cosmétique selon la revendication 1, **caractérisé en ce qu'**à côté de la gomme de gellan en tant que gélifiant sont présents ceux du groupe choisi parmi le carbomère, la gomme de xanthane, le carraghénane, la gomme d'acacia, la gomme de guar, l'agar-agar, l'alginate, le polyacétal vinylique (PVA), la polyvinylpyrrolidone (PVP) et des mélanges de ceux-ci.

8. Produit cosmétique selon la revendication 1, **caractérisé en ce que** les sels inorganiques sont choisis dans le groupe qui se compose des sels de sodium, de potassium, de magnésium, de calcium, de manganèse (II), de baryum, de fer (II), de fer (III) et des mélanges de ceux-ci.

9. Produit cosmétique selon la revendication 1, **caractérisé en ce que** le produit est un crayon ayant un diamètre de 5-15 mm et une valeur de pénétration de 15 à 30.

10. Produit cosmétique selon la revendication 1, **caractérisé en ce que** le produit est un crayon ayant un diamètre > 15 mm et une valeur de pénétration de 31 à 66.

11. Produit cosmétique selon la revendication 1, **caractérisé en ce que** le produit est un fond de teint compact ayant une valeur de pénétration de 42 à 52.
